# EUROPEAN PATENT APPLICATION

(11) **EP 0 919 224 A1**
(43) Date of publication of application: **02.06.1999**
(21) Application number: 98119882.3
(22) Date of filing: 20.10.1998
(51) Int. Cl.: A61K 7/48

(54) **Cleaning nose oil**

(30) Priority: 20.10.1997 EP 97118150
(71) Applicant: Chemisch Adviesbureau Drs. J.C.P. Schreuder B.V., NL-3740 AK Baarn (NL)
(72) Inventor: Schreuder, Johannes Carolus Petrus, 3740 AK Baarn (NL)
(74) Representative: DIEHL GLAESER HILTL & PARTNER

(57) **Abstract**

There is provided a cleaning nose oil for human beings, comprising at least a naturally occurring, homogeneous liquid branched hydrocarbon oil having from 20 to 40 carbon atoms.

## Description

The invention relates to a cleaning nose oil for human beings. In particular the invention relates to an oil for cleaning and treating the mucosa of the nose of human beings and in particular of babies with a cold.

Especially babies of up to about one year old are rather prone to colds and can then be helpless sniffling.

On the other hand, such babies will easily start or continue crying, when their nose in such event is cleaned up with strange smelling or irritating cleaning liquids or creams, while the application of non-natural cleaning agents will moreover enhance the risk of further irritation due to disturbance of the natural biological equilibrium of the vulnerable internal and external nose tissue and subsequently to chapped, sore and/or even inflamed skin parts.

Therefore one object of the invention is to provide an odorless, tasteless, stable and inert homogeneous nose cleaning liquid, which does not disturb the natural biological equilibrium of the internal and external vulnerable nose tissue, which facilitates an almost immediate relief and healing, and which can easily be applied, e.g. by means of cotton pads or a tuft or wad of cotton or by means of a nebulizer or spray.

It has now surprisingly been found that as such a cleaning liquid aimed at can be used a naturally occurring homogeneous liquid branched hydrocarbon oil, having from 20 to 40 carbon atoms and preferably from 26 to 32 carbon atoms.

Accordingly, the present invention relates to a cleaning and careing nose oil, comprising at least a naturally occurring homogeneous liquid branched hydrocarbon oil, having from 20 to 40 carbon atoms and preferably from 26 to 32 carbon atoms.

Preferably the branches attached to the main carbon chain in said oil contain 1 to 2 carbon atoms each.

According to a most preferred embodiment of the present invention the cleaning nose oil comprises at least squalane.

Said branched hydrocarbon oil and most preferably squalane will preferably be applied in a sterilized or pasteurized condition and packed in bottles or tins, provided with a germ-excluding screw/locking cap.

Another aspect of the present invention is formed by the use of the cleaning nose oil, comprising putting on the nose mucous tissue an amount from 0.1 to 0.5 grams by means of a cotton pad, a cotton wad or a nebulizer spray.

Preferably amounts of from 0.2 to 0.3 grams are applied per cleaning operation.

It has been found that such branched hydrocarbon oil not only enables the cleaning of the nose tissue inclusive the removal of hard and dry scabs or crusts, but also acts as a natural emollient, which facilitates an almost immediate relief and opening of the nose respiration tract, when applied in the hereinbefore specified small portions.

According to a preferred embodiment of said application, a plastic nebulizer is used, which applies the branched hydrocarbon oil by hand pressing or by means of a non-toxic inert propellant gas such as pressurized air or oxygen.

It will be appreciated that the hereinbefore specified branched hydrocarbon oil can be mixed with minor amounts of additional auxiliaries or medicaments, such as vitamins and/or antibiotics and/or anti-inflammable agents.

Accordingly the present invention also relates to fixed amounts of cleaning nose oil, comprising at least a naturally occurring homogeneous liquid branched hydrocarbon oil having from 20 to 40 carbon atoms and preferably from 26 to 32 carbon atoms and most preferably squalane, optionally mixed with small amounts (less than 5 wt% of the complete composition) of one or more additional auxiliaries.

## Claims

1. Cleaning nose oil for human beings, comprising at least a naturally occurring homogeneous liquid branched hydrocarbon oil having from 20 to 40 carbon atoms.

2. Cleaning nose oil according to claim 1, characterized in that the liquid branched hydrocarbon oil has 26 to 32 carbon atoms.

3. Cleaning nose oil according to claims 1 or 2, characterized in that the branches attached to the main carbon chain in the liquid branched hydrocarbon oil have 1 or 2 carbon atoms each.

4. Cleaning nose oil according to any of claims 1 to 3, characterized in that the liquid branched hydrocarbon oil is squalane.

5. Cleaning nose oil according to any of claims 1 to 4, characterized in that it is packed in a sterilized or pasteurized bottle or tin.

6. Use of the cleaning nose oil according to any of claims 1 to 5 for cleaning the nose of human beings.

7. Use according to claim 6, which comprises putting on the nose mucous tissue an amount from 0.1 to 0.5 grams by means of a cotton pad or a nebulizer or spray.

8. Use according to claim 7, characterized in that amount of from 0.2 to 0.3 grams of the cleaning nose oil are put on the nose mucuos tissue.
